# FASCICULE DE BREVET EUROPEEN

(11) **EP 0 352 689 B1**
(45) Date de publication et mention de la délivrance du brevet: **13.01.1993**
(21) Numéro de dépôt: 89113559.2
(22) Date de dépôt: 24.07.1989
(51) Int. Cl.: G01N 21/07

(54) **Dispositif pour la réalisation d'analyses biologiques par détection immuno-enzymatique d'anticorps ou d'antigènes dans un sérum**
Vorrichtung für biologische Analysen mittels Enzymimmun-Test von Antikörpern oder Antigenen in einem Serum
Device for biological analyses by enzymimmunassay detection of antibodies or antigens in a serum

(30) Priorité: 28.07.1988 FR 8810211
(43) Date de publication de la demande: 31.01.1990
(73) Titulaire: Guigan, Jean, F-75005 Paris (FR)
(72) Inventeur: Guigan, Jean, F-75005 Paris (FR)
(74) Mandataire: Weinmiller, Jürgen

(56) Documents cités:
- EP-A- 0 166 933
- EP-A- 0 192 968
- US-A- 3 726 645

## Description

La présente invention concerne un dispositif pour la réalisation d'analyses biologiques par détection immuno-enzymatique d'anticorps ou d'antigènes dans un sérum.

Une méthode immuno-enzymatique connue en phase solide indirecte comprend schématiquement les phases suivantes :
- Introduction du sérum dont on cherche à détecter et quantifier les anticorps dans un puits cylindrique en matière plastique de petite dimension (1 cm de hauteur, et 5 mm de diamètre environ) ; la surface interne de ce puits est initialement revêtue d'antigènes. On attend que les anticorps se soient fixés sur les antigènes.
- Premier lavage éliminant le sérum, et introduction d'un liquide conjugué marqué par une enzyme qui se fixe à son tour sur les liaisons antigènes-anticorps.
- Second lavage destiné à éliminer le conjugué en excès.

A partir de ce stade, il existe deux modes opératoires différents :
Le premier utilise la colorimétrie : on introduit un substrat incolore qui développe une réaction colorée par l'action de l'enzyme résiduelle et on lit au photomètre.

Le second utilise la luminescence : on introduit un réactif appelé "Signal" et la lecture est alors effectuée avec un analyseur de luminescence.

Ces mesures permettent de déterminer la quantité d'anticorps ou d'antigènes présents dans le sérum du patient.

La fixation des anticorps sur les antigènes, dans les conditions classiques d'application de la méthode, peut mettre 5 à 6 heures à se développer, cette durée étant nécessaire pour qu'un nombre suffisant d'anticorps se fixe sur les antigènes. En effet, les premières liaisons antigènes-anticorps encombrent la surface du puits, rendant son accès plus difficile aux anticorps suivants.

La présente invention a pour but de mettre en oeuvre un dispositif permettant de réduire considérablement la durée globale de l'analyse, et de limiter les manipulations indispensables lors de la mise en oeuvre de la méthode antérieure.

La présente invention a pour objet un dispositif pour la réalisation d'analyses biologiques par détection immuno-enzymatique d'anticorps ou d'antigènes dans un sérum, caractérisé par le fait qu'il comporte un dispositif de traitement simultané d'une pluralité de cartouches, l'ensemble étant disposé dans une enceinte thermostatée, ledit dispositif de traitement comprenant :
- un moyeu associé à un moteur à codeur angulaire permettant la gestion des cycles de rotation du moyeu et susceptible de lui imprimer un mouvement rapide pour une centrifugation ou un mouvement lent pas à pas,
- une pluralité d'ascenseurs porte-cartouche fixés radialement sur le moyeu et des moyens pour les faire passer chacun d'une position haute de chargement d'une cartouche à une position basse de travail, chacun de ces ascenseurs présentant une face périphérique ouverte, une face supérieure et une face inférieure très échancrées, chaque ascenseur comprenant des moyens débrayables pour bloquer radialement sa cartouche en cas de centrifugation,
- une pluralité de calibres de lecture fixés audit moyeu au droit respectivement des faces périphériques desdits ascenseurs, dispositif caractérisé également par le fait que chaque cartouche de forme générale rectangulaire comprend un fond en matière plastique thermoformé recouvert d'une feuille transparente mince en matière plastique et formant ainsi :
- une cuve réceptrice disposée en position centrale reliée par un conduit à une cuve de stockage de sérum située à l'extrémité de la cartouche destinée à se trouver du côté dudit moyeu,
- entre la cuve réceptrice et ladite cuve de stockage de sérum, au moins deux cuves reliées par des conduits à ladite cuve réceptrice et destinées à contenir respectivement une poche de liquide conjugué et une poche de liquide substrat, ladite cuve réceptrice présentant un petit trou d'évacuation de liquide dans son fond et contenant un puits sensiblement cylindrique fermé par un couvercle, rendu mobile en rotation autour d'un axe vertical, dont une portion de paroi latérale forme un bec verseur ou récepteur de tous les liquides susceptibles de passer dans les conduits précités, et qui contient un écouvillon à grande surface spécifique, mobile en rotation autour d'un axe vertical indépendamment dudit puits, les brins dudit écouvillon étant porteurs d'antigènes,
- une poche souple destinée à contenir un liquide bloquant ou diluant, reliée par un conduit à ladite cuve réceptrice, et située à proximité de la face périphérique ouverte de l'ascenseur correspondant, ledit conduit étant obturé par un opercule frangible,

ledit dispositif de traitement comprenant en outre au moins un module périphérique de lecture situé sur la trajectoire desdits calibres, et un module situé au-dessus de la trajectoire desdites cartouches et muni de moyens de perçage desdites poches contenant respectivement le conjugué et le substrat ainsi que des moyens de rupture dudit opercule frangible.

De préférence, ledit dispositif de traitement est associé à au moins une pipette de remplissage automatique en sérum des cuves correspondantes desdites cartouches.

Ledit dispositif de traitement comporte en outre avantageusement un module de chargement automatique d'une cartouche dans chaque ascenseur, ainsi qu'un module de déchargement automatique.

Selon un perfectionnement, ledit dispositif de traitement comporte un module muni de moyens pour s'appliquer contre ladite poche souple et assurer une homogénéisation des liquides qu'elle contient.

Selon un mode de réalisation avantageux, l'axe vertical dudit écouvillon est solidaire d'une petite étoile métallique susceptible de coopérer avec un module dudit dispositif de traitement muni d'un électro-aimant situé au droit de la trajectoire dudit axe vertical, afin de faire tourner régulièrement ledit écouvillon sur lui-même. Ce même module comporte en outre un électro-aimant susceptible de coopérer avec le couvercle ou le fond dudit puits extérieurement métallisé, afin de faire tourner ledit puits autour de son axe vers une première position où son bec verseur est orienté en direction dudit trou d'évacuation et vers une seconde position où son bec verseur est orienté vers le conduit aboutissant à ladite poche souple.

Par ailleurs, ledit module situé au dessus de la trajectoire desdites cartouches comporte des marteaux commandables par des électro-aimants, susceptibles d'aplatir lesdites poches, un organe coupant étant prévu entre le fond de ces poches et la cuve qui les contient ; ce module comporte également un marteau commandable par un électro-aimant susceptible de casser ledit opercule frangible.

De manière avantageuse, chaque calibre de lecture est une pièce transparente présentant sensiblement la forme d'une gouttière de manière à bloquer radialement ladite poche souple, lesdits moyens de blocage de ladite cartouche dans lesdits ascenseurs étant alors débrayés, ladite gouttière ayant deux parois latérales horizontales d'écartement calibré contre lesquelles ladite poche souple vient se plaquer au cours d'une centrifugation.

D'autres caractéristiques et avantages de la présente invention apparaîtront au cours de la description suivante d'un mode de réalisation donné à titre illustratif mais nullement limitatif.

Dans le dessin annexé :
- La figure 1 est une vue partielle en perspective semi-éclatée d'une cartouche d'analyse selon l'invention.
- La figure 2 est une vue en perspective de la cartouche de la figure 1.
- La figure 3 est une vue en perspective éclatée du puits appartenant à la cartouche des figures 1 et 2.
- Les figures 4 à 6 sont des vues de dessus très schématiques de la cartouche précédente montrant le puits de la figure 3 dans trois positions différentes.
- La figure 7 est une vue schématique de dessus du dispositif de traitement des cartouches.
- La figure 8 est une vue schématique en coupe selon la ligne VIII-VIII de la figure 7.
- La figure 9 est une vue schématique en perspective d'un ascenseur porte-cartouche appartenant au dispositif des figures 7 et 8.
- La figure 10 est une vue latérale schématique d'un premier système de rappel de cartouche appartenant à l'ascenseur porte-cartouche de la figure 9.
- La figure 11 est une vue latérale schématique d'un second système de rappel de cartouche appartenant à l'ascenseur porte-cartouche de la figure 9.
- La figure 12 est une vue schématique en perspective d'un module appartenant au dispositif de traitement précédent.
- Les figures 13 à 30 montrent schématiquement les différentes étapes de la réaction qui se produit dans une cartouche.

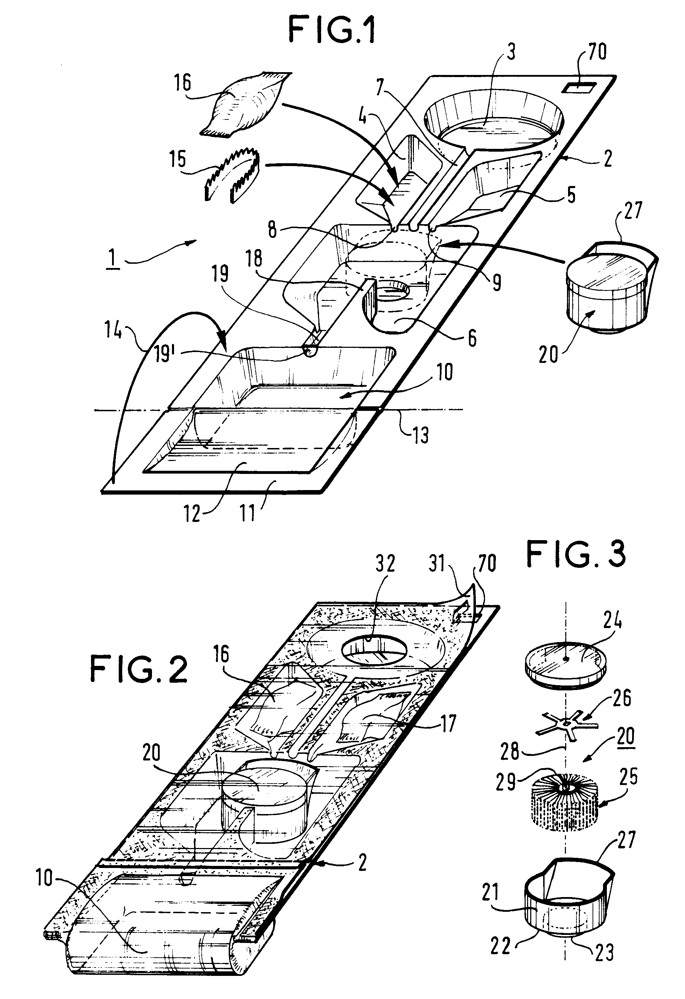

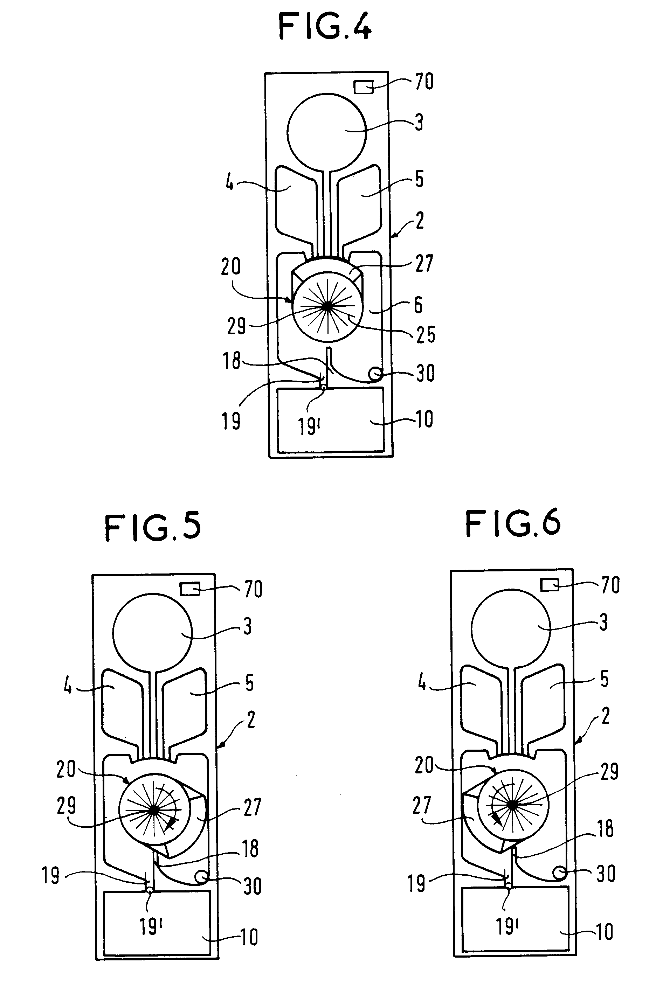

Les figures 1, 2, 3 illustrent en perspective la cartouche 1 qui remplace le petit tube de l'art antérieur. Une telle cartouche est formée de la manière suivante (voir figure 1). Elle comporte un fond 2 en matière plastique thermoformable, par exemple en polystyrène, de forme globalement rectangulaire et comportant plusieurs cuves de stockage :
- Une cuve 3 de stockage de sérum située à une première extrémité.
- Une cuve 4 de stockage d'un conjugué marqué par une enzyme.
- Une cuve 5 de stockage d'un substrat hydrolysable par ladite enzyme.
- Une cuve réceptrice 6 communiquant respectivement avec lesdites cuves 3, 4 et 5 par trois conduits 7, 8, 9. Outre le puits 20 qui va être décrit en détail plus loin, la cuve réceptrice 6 présente du côté opposé aux trois conduits 7, 8, 9 deux zones limitées par une paroi 18, la première zone communiquant avec la poche suivante 10 par un conduit 19, et la seconde zone communiquant avec l'extérieur par un trou 30 ménagé dans le fond 2 et visible dans les figures 4 à 6.
- Une poche d'analyse 10 formée par rabattement sur le fond 2 d'une partie 11 thermoformée en creux 12 et rabattue autour d'un axe 13 suivant la flèche 14. La poche d'analyse 10 plus nettement visible dans la figure 2 est fermée par thermosoudage. Dans l'état initial, la communication entre la poche 10 et le conduit 19 est obturée par un opercule frangible 19′. Avant sa fermeture complète, la poche 10 est remplie partiellement d'un liquide bloquant ou diluant.

Les cuves 4 et 5 contiennent initialement sur leur fond un organe coupant schématiquement illustré en 15 et une poche contenant le conjugué ou le substrat. On voit dans la figure 2 la poche de conjugué 16 et la poche de substrat 17.

La cuve réceptrice 6 contient un élément fondamental de l'invention qui est le puits 20 en matière plastique. Il est constitué (voir figure 3) d'un corps creux 21 muni d'un fond 22 présentant une protubérance 23 destinée à s'emboîter dans le fond 2 de la cartouche 1 ; une portion de la paroi latérale du corps creux 21 forme un bec verseur 27 normalement orienté vers la cuve 3 de stockage de sérum, comme illustré dans les figures 1 et 2. La forme du bec 27 est adaptée de manière à venir juste au-dessous des conduits 7, 8, 9 pour recueillir tout liquide venant de ces conduits. Le fond 22, ou le couvercle 24 du corps 21, est métallisé extérieurement de manière à coopérer avec un électro-aimant permettant de faire tourner le puits autour de son axe 28, jusqu'à l'une ou l'autre des positions illustrées respectivement par les figures 5 et 6. Dans la figure 5, le bec verseur 27 est orienté vers le trou 30 ; dans la figure 6, le bec verseur 27 est orienté vers le conduit 19. Dans les deux cas la paroi du bec 27 et la paroi 18 coopèrent de manière à délimiter deux zones de déversement distinctes.

La partie essentielle du puits 20 (cf figure 3) est un petit écouvillon 25, d'axe 29, solidaire d'une petite étoile métallique 26. Comme on le verra plus loin, cette petite étoile 26, sous l'action d'un électro-aimant, permet de faire tourner régulièrement l'écouvillon 25 autour de son axe 29.

Lorsque tout est en place dans les différentes cuves du fond 2, on le recouvre d'une mince feuille en Surlyn 31 (marque de la société DU PONT DE NEMOURS), présentant une ouverture 32 au droit de la cuve 3 de stockage de sérum, et qui est thermosoudée sur les parois non thermoformés du fond 2 (cf figure 2).

A titre d'exemple, la cartouche mesure 50 mm de long, 15 mm de large et un peu plus de 10 mm de hauteur. Le puits 20 présente un diamètre intérieur de 7 mm et une hauteur de 10 mm. La feuille de Surlyn a une épaisseur de l'ordre de 40 microns.
Les cuves 3, 4 et 5 sont destinées à recevoir respectivement :
- 100 à 200 microlitres de sérum
- 100 microlitres de conjugué
- 100 microlitres de substrat.

La poche 10 peut recevoir 250 microlitres de liquide bloquant ou autre.

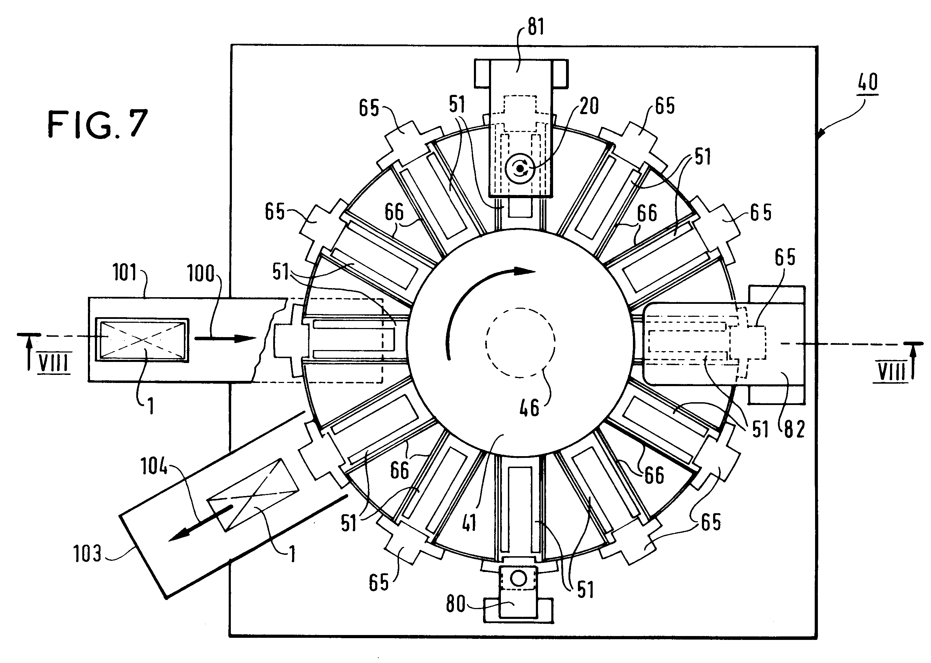

La cartouche précédemment définie et munie de ses moyens d'identification est destinée à être traitée dans un dispositif 40 illustré à l'aide des figures 7 à 12 qui constitue un mini-laboratoire. Il se présente sous la forme générale d'un boîtier parallélépipédique 50 dont la base a, par exemple, pour dimensions transversales 300 mm x 300 mm et pour hauteur 200 mm environ. Il est placé dans une enceinte thermostatée, de préférence à 37°C.

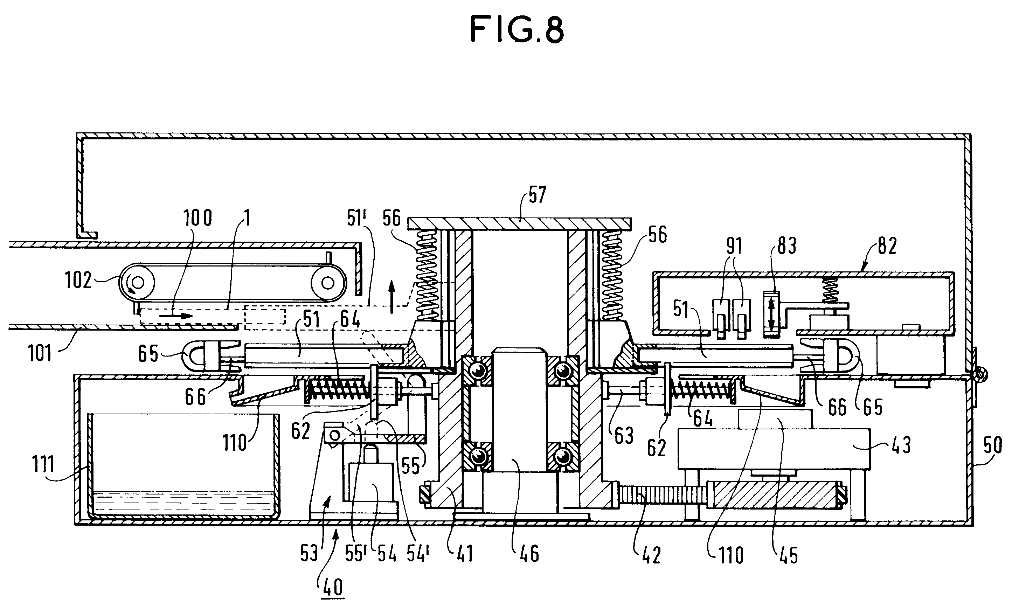

Les principaux sous-ensembles de ce dispositif 40 sont les suivants (cf figures 7 et 8) :
Un moyeu 41 peut être entraîné en rotation autour d'un axe 46 par un moteur 43 avec un système de transmission schématisé 42 comportant une courroie crantée et deux pignons à dents. Il s'agit d'un moteur à courant continu associé à un codeur angulaire 45 permettant la gestion des cycles de rotation. Le moyeu 41 est solidarisé à douze ascenseurs porte-cartouche tels que 51, répartis régulièrement radialement autour de lui. Chaque ascenseur porte-cartouche 51 est associé à un système de commande 53, destiné à faire passer l'ascenseur porte-cartouche 51 d'une position 51′ levée illustrée en pointillés, à une position basse illustrée en traits pleins. Le système de commande 53 peut être constitué d'un électro-aimant commandable 54 associé à un levier 55. On a illustré en pointillés les positions 54′ et 55′ de l'électro-aimant et du levier correspondant à la position 51′ de l'ascenseur porte-cartouche 51. Des ressorts de rappel 56 sont prévus entre les ascenseurs 51 et la face supérieure 57 du moyeu 41.

Quand l'ascenseur est en position 51′, on peut y introduire manuellement une cartouche 1 suivant la flèche 100 comme indiqué dans la figure 8 ; de préférence cette introduction est effectuée par un module de chargement automatique 101 (avec entraînement schématisé 102). De même un module de déchargement automatique 103 dans la direction schématisée par la flèche 104 est illustré dans la figure 7.

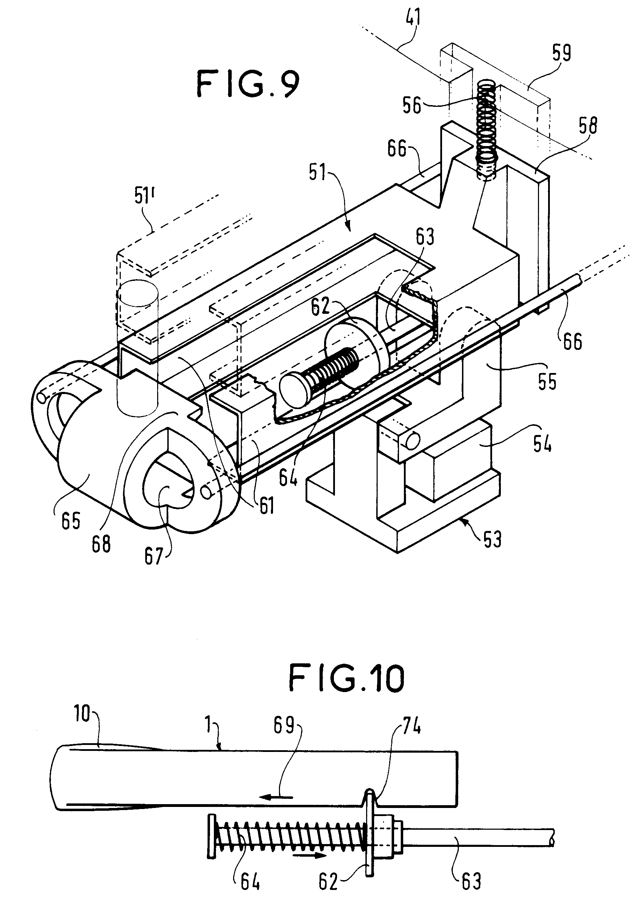

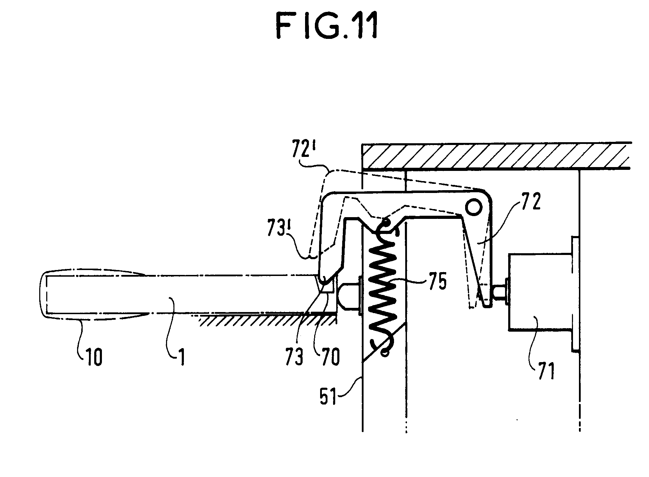

On voit plus précisément dans la figure 9 un ascenseur 51 présentant une partie en T 58 guidée dans une rainure correspondante 59 du moyeu 41. Du côté opposé, l'ascenseur 51 a sa face d'entrée ouverte, et sur ses parties latérales il est formé de rails 61 à section en U, sa face supérieure étant également largement ouverte. Il en est de même pour son fond d'où dépasse un disque 62 d'indexation de cartouche susceptible de coulisser sur une tige 63 solidaire du moyeu 41 et coopérant avec un ressort de rappel 64 bien visible dans la figure 10. On voit également dans cette figure une encoche 74 prévue dans la cartouche 1 où s'introduit le disque 62. Ce système est susceptible d'entraîner la cartouche 1 vers la face de sortie de l'ascenseur 51 dans le sens de la flèche 69. Au contraire le système, illustré dans la figure 11 et coopérant avec un orifice 70 de la cartouche 1, est destiné à la retenir vers le centre du moyeu quand il est dans la position illustrée en traits pleins. Il s'agit d'un bras mobile 72 associé à un ressort de rappel 75 et actionné par un électro-aimant commandable 71. Le bras 72 peut passer à la position 72′ et son extrémité 73 insérée dans l'orifice 70 passe alors en 73′ et quitte cet orifice. La cartouche 1 est alors prise en charge par le système de la figure 10 qui la guide radialement vers la périphérie du dispositif 40.

Sur la partie périphérique du dispositif 40 et au droit de chaque face d'entrée d'un ascenseur 51 est disposé un calibre de lecture optique 65 solidarisé au moyeu 41, par exemple par l'intermédiaire de tiges 66. Chaque calibre 65 est une pièce (voir figures 7 et 9) sensiblement en forme de gouttière ajourée avec un intervalle parfaitement calibré entre les parois latérales 67 et 68. Il s'agit par exemple d'une pièce injectée en polycarbonate, parfaitement transparente.

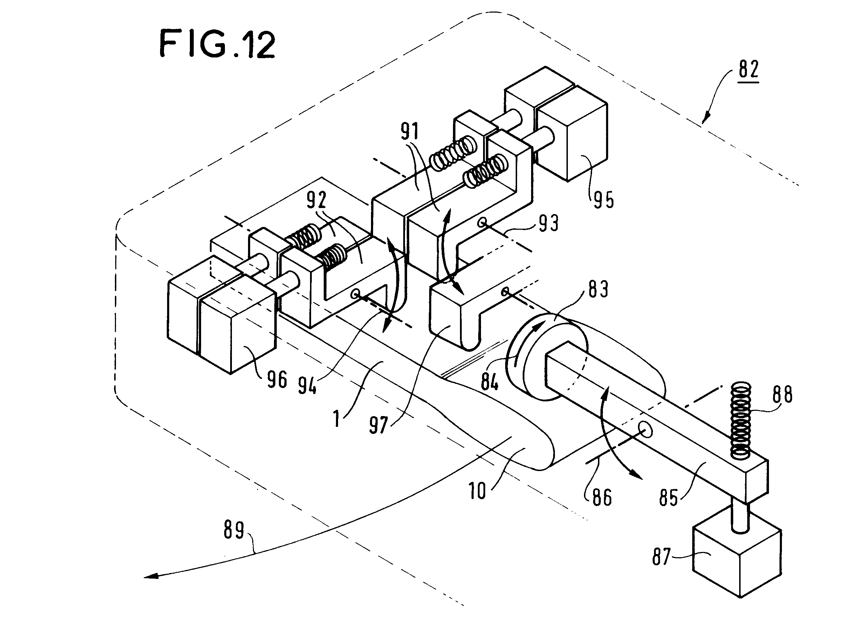

On a schématisé dans la figure 7 un module 80 destiné à la lecture des réactions par colorimétrie, ou luminescence ou autre méthode adaptée à travers les calibres 65. Dans cette figure apparaissent également un module 81, où sont situés des électro-aimants destinés à effectuer les manoeuvres de rotation du puits 20 des cartouches 1, et un module 82 illustré plus en détail dans la figure 12. Ce module 82 comporte tout d'abord une roulette 83, mobile selon le sens de la flèche 84 et solidaire d'un bras 85 mobile en rotation autour d'un axe 86 permettant un escamotage de la roulette 83. Le bras est commandé par un électro-aimant 87 et un ressort de rappel 88 fixé au module 82. La roulette 83 se situe au droit de l'emplacement de la poche 10. Le module 82 comporte également des marteaux 91 et 92 rendus mobiles en rotation respectivement autour des axes 93 et 94 par des électro-aimants 95 et 96. Ces marteaux sont situés au droit des poches de réactifs 16 et 17 munies de leur organe coupant 15. Un autre marteau 97 est situé au droit de l'emplacement de l'opercule frangible 19′ afin de le rompre quand cela est nécessaire.

Le dispositif 40 comporte enfin (voir figure 8) sous les ascenseurs 51 un réservoir annulaire fixe collecteur d'égouttures 110, relié à un réservoir amovible 111.

On va décrire ci-dessous les différentes phases de mise en oeuvre d'une cartouche 1 dans le dispositif de traitement 40.

Lorsque l'on introduit la cartouche 1 par l'intermédiaire du module de chargement automatique 101 dans un ascenseur porte-cartouche en position 51′ (figure 8), elle est dans l'état illustré par la figure 2 avec les deux poches 16 et 17 en place, du liquide bloquant 120 dans la poche souple 10 et dans le puits 20 l'écouvillon 25 sur les brins duquel sont fixés des antigènes. La surface spécifique de l'écouvillon 25 étant très supérieure à celle du puits de l'art antérieur, le puits 20 contient une quantité d'antigènes répartis sur une surface beaucoup plus considérable.

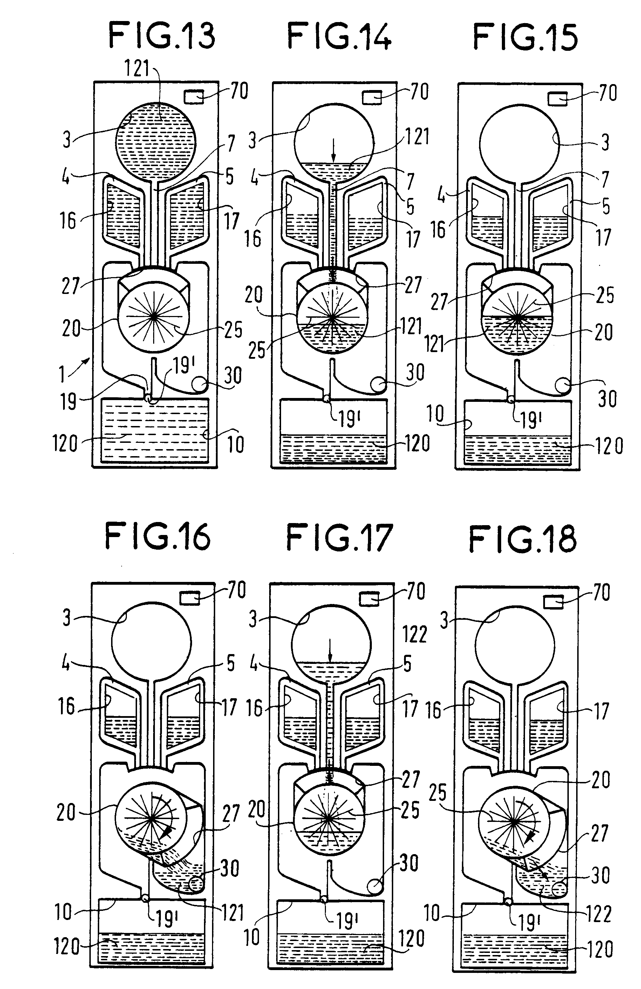

Au moment de son chargement la cartouche 1 se trouve mise en place vis-à-vis des systèmes illustrés dans les figures 10 et 11 grâce à son orifice 70 et à son encoche 74 (figure 10).
Lorsque l'ascenseur porte-cartouche est revenu en position 51, le moteur 43 fait tourner le moyeu 41 d'un douzième de tour afin que la cartouche suivante puisse être chargée (figures 7 et 8). Au cours de l'opération de chargement de la seconde cartouche, il est avantageux d'introduire du sérum 121 dans la cuve 3 de la première cartouche. Ceci peut être fait automatiquement par exemple en utilisant la pipette décrite dans le brevet français du demandeur n°87 15688. La cartouche se trouve alors dans l'état illustré par la figure 13.

Lorsque l'ensemble des cartouches est chargé, on effectue par rotation du moyeu 41 une centrifugation de manière à faire passer le sérum 121 par le conduit 7 dans le puits 20 (figures 14 et 15). Le sérum se trouve alors au contact des antigènes et les anticorps éventuels se fixent sur les antigènes. Cette opération prend quelques dizaines de minutes. Pendant ce temps, le moyeu 41 tourne régulièrement de manière que les cartouches s'arrêtent successivement sous le module 81 (figure 7) dont l'électro-aimant fait tourner d'un cinquième de tour la petite étoile métallique 26, et par là-même l'écouvillon 25, toujours immergé environ à moitié. Grâce à cette rotation, chaque brin de l'écouvillon 25 passe de la position immergée qui lui permet de collecter des anticorps à la position émergée dans laquelle il se débarrasse totalement de sérum appauvri en anticorps. Ce mouvement d'agitation du sérum et la grande surface spécifique de l'écouvillon permettent d'aboutir à une fixation antigènes-anticorps en un temps considérablement réduit par rapport aux procédés antérieurs (cf. figure 15).

Après quelques dizaines de minutes, la cartouche 1 passe au niveau du module 81 dont un électro-aimant agit de manière à faire tourner le puits 20 comme illustré dans la figure 16, le bec verseur 27 étant tourné vers le trou 30 par lequel est évacué le sérum 121 appauvri. Ce dernier aboutit dans le réservoir annulaire 110 (figure 8). Après le vidage, la cartouche 1, toujours mise en place sous le module 81, voit son puits 20 revenir en position initiale. Par l'utilisation d'une pipette non illustrée, quelques dizaines de microlitres d'eau de rinçage 122 sont introduits dans la cuve 3. La cartouche subit alors une centrifugation (cf figure 17) et l'eau 122 aboutit dans le puits 20.

Le moyeu 41 continue à tourner et, à chaque passage au niveau du module 81, l'écouvillon tourne sur lui-même de manière que tous les brins soient rincés. Après quelques minutes, une station de la cartouche sous le module 81 permet, comme dans la figure 16, d'évacuer l'eau de rinçage 122 par rotation du puits 20 vers le trou 30 (cf figure 18).

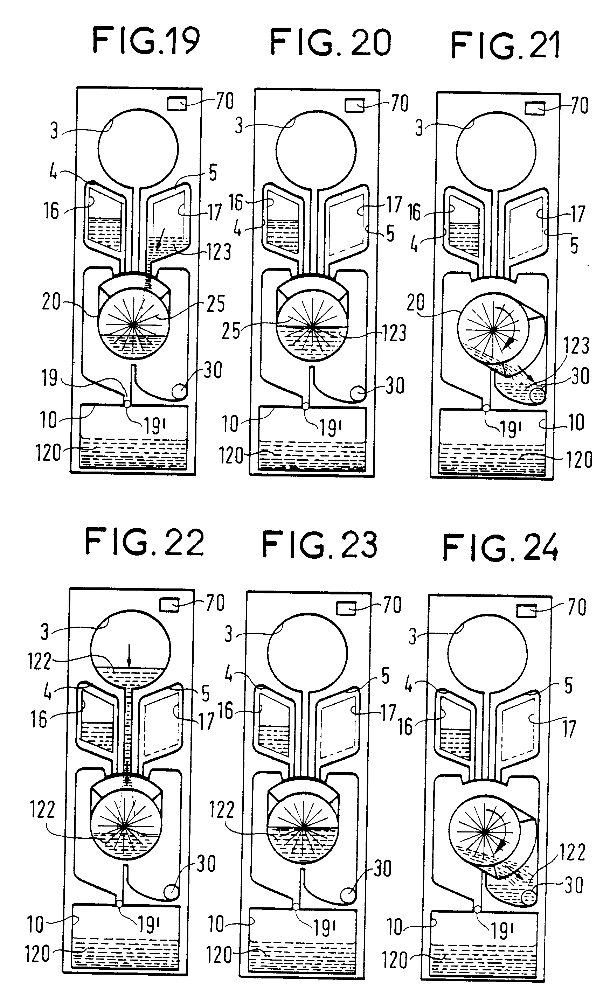

La cartouche 1 passe ensuite sous le module 82 (cf figure 12) et l'électro-aimant 95 agit sur les marteaux 91 qui viennent comprimer la poche 17 contre l'organe coupant 5. La poche 17 se trouve crevée et le liquide conjugué marqué par une enzyme 123 peut s'écouler. Grâce à une centrifugation il parvient dans le puits 20 et se trouve au contact des brins de l'écouvillon. (cf figures 19 et 20). Là encore, pendant quelques minutes, l'écouvillon tourne sur lui-même pour que le conjugué 123 puisse se fixer sur toutes les liaisons antigènes-anticorps.

Puis, le conjugué 123 est éliminé par rotation du puits 20 vers le trou 30 (cf figure 21).

On renouvelle l'opération de rinçage des figures 17 et 18, et les phases successives apparaissent dans les figures 22 à 24.

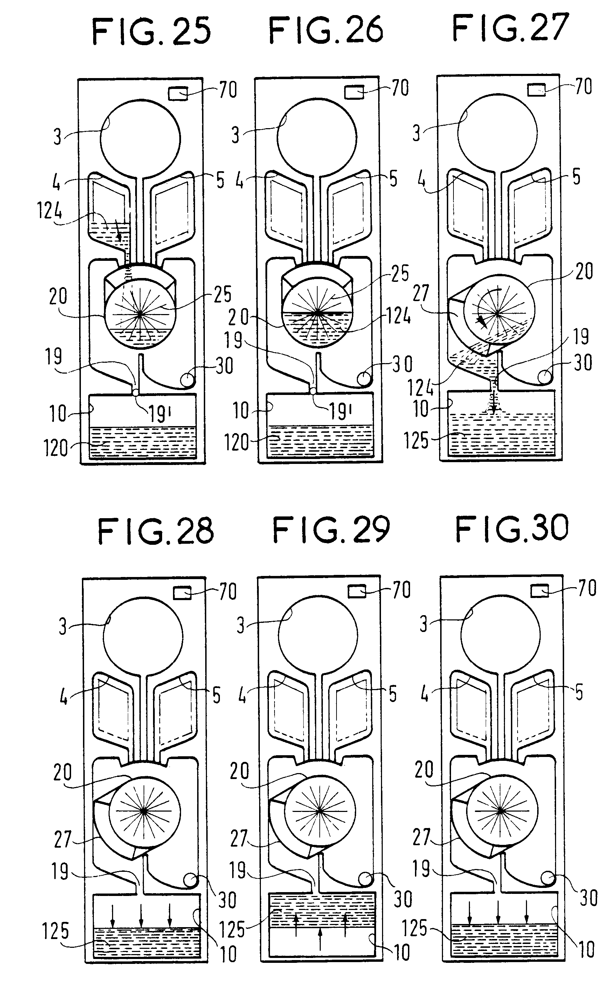

Lorsque la cartouche 1 repasse sous le module 82 (cf figure 12), ce sont les marteaux 92 qui agissent sur la poche 16 qui se crève comme la poche 17 et laisse échapper le substrat 124. Une centrifugation met en contact ce substrat avec l'écouvillon 25 (cf figures 25 et 26). Une réaction colorée va se développer pendant quelques minutes au cours desquelles l'écouvillon 25 tourne sur lui-même. Lors d'un passage au niveau du module 82 (cf figure 12), le marteau 97 agit sur l'opercule frangible 19′ et libère la communication entre la cuve 6 et la poche souple 10. Après une rotation du puits 20 de manière que son bec 27 soit orienté vers le conduit 19, et une nouvelle centrifugation, on fait passer le liquide 124 dans la poche 10, où la réaction se trouve fixée (cf figure 27).
Toutes les opérations successives de centrifugation ont l'avantage de supprimer tout effet de mouillage des brins de l'écouvillon et de les débarrasser successivement de tous les liquides en excès introduits dans le puits.

Lors du passage de la cartouche 1 au niveau du module 82 (cf figure 12), la roulette 83, appliquée contre la poche 10 par action de l'électroaimant 87 sur le bras 85, tourne sur elle-même et homogénéise le mélange 125 (cf figures 28 à 30). La cartouche 1 est alors prête à l'examen.

L'électro-aimant de la figure 11 va alors agir sur le bras 72 qui va passer en position 72′ et libérer la cartouche 1 rappelée alors vers la périphérie du dispositif par le ressort 64 de la figure 10. Sous l'effet de la centrifugation la pochette souple 10 partiellement remplie de liquide 25 va venir se plaquer contre les parois planes d'écartement calibré 67 et 68 du calibre 25 qui lui est associé (cf figure 9). Une mesure par colorimétrie ou luminescence, ou autre, sera effectuée au passage de la cartouche 1 sous le module de mesure 80.

L'ensemble des opérations du dispositif 40 est piloté par un ordinateur programmé. Le dispositif 40 associé aux cartouches 1 permet donc d'effectuer automatiquement de manière fiable et précise toutes les opérations nécessaires à une détection immuno-enzymatique et dans un temps beaucoup plus bref que les dispositifs connus. En effet la fixation des anticorps sur les antigènes ne demande plus que quelques dizaines de minutes et les autres étapes ne demandent chacune que quelques minutes.

Bien entendu, l'invention n'est pas limitée au mode de réalisation qui vient d'être décrit. On pourra remplacer tout moyen par un moyen équivalent sans sortir du cadre de l'invention. Ainsi par exemple l'écouvillon pourrait être remplacé par un élément à surface développée équivalente.

Pour la commodité du dessin, on a illustré douze ascenseurs porte-cartouche, mais les dimensions mentionnées permettent d'en loger dix-huit, ce nombre n'étant également qu'un exemple de mise en oeuvre.

## Revendications

1. Dispositif pour la réalisation d'analyses biologiques par détection immuno-enzymatique d'anticorps ou d'antigènes dans un sérum, caractérisé par le fait qu'il comporte un dispositif de traitement simultané d'une pluralité de cartouches (1), l'ensemble étant disposé dans une enceinte thermostatée, ledit dispositif de traitement comprenant :
- un moyeu (41) associé à un moteur (43) à codeur angulaire permettant la gestion des cycles de rotation du moyeu et susceptible de lui imprimer un mouvement rapide pour une centrifugation ou un mouvement lent pas à pas,
- une pluralité d'ascenseurs porte-cartouche (51) fixés radialement sur le moyeu (41) et des moyens pour les faire passer chacun d'une position haute de chargement d'une cartouche à une position basse de travail, chacun de ces ascenseurs présentant une face périphérique ouverte, une face supérieure et une face inférieure très échancrées, chaque ascenseur comprenant des moyens débrayables pour bloquer radialement sa cartouche en cas de centrifugation,
- une pluralité de calibres de lecture (65) fixés audit moyeu au droit respectivement des faces périphériques desdits ascenseurs, dispositif caractérisé également par le fait que chaque cartouche (1) de forme générale rectangulaire comprend un fond (2) en matière plastique thermoformé recouvert d'une feuille transparente mince en matière plastique et formant ainsi :
- une cuve réceptrice (6) disposée en position centrale reliée par un conduit (7) à une cuve de stockage de sérum (3) située à l'extrémité de la cartouche destinée à se trouver du côté dudit moyeu,
- entre la cuve réceptrice et ladite cuve de stockage de sérum, au moins deux cuves (4, 5) reliées par des conduits (8, 9) à ladite cuve réceptrice (6) et destinées à contenir respectivement une poche de liquide conjugué et une poche de liquide substrat, ladite cuve réceptrice présentant un petit trou d'évacuation (30) de liquide dans son fond et contenant un puits (20) sensiblement cylindrique fermé par un couvercle, rendu mobile en rotation autour d'un axe vertical (28), dont une portion de paroi latérale forme un bec verseur (27) ou récepteur de tous les liquides susceptibles de passer dans les conduits précités, et qui contient un écouvillon (25) à grande surface spécifique, mobile en rotation autour d'un axe vertical (29) indépendamment dudit puits, les brins dudit écouvillon étant porteurs d'antigènes,
- une poche souple (10) destinée à contenir un liquide bloquant ou diluant, reliée par un conduit (19) à ladite cuve réceptrice, et située à proximité de la face périphérique ouverte de l'ascenseur correspondant, ledit conduit étant obturé par un opercule frangible (19′),
ledit dispositif de traitement comprenant en outre au moins un module périphérique de lecture (80) situé sur la trajectoire desdits calibres (65), et un module (82) situé au-dessus de la trajectoire desdites cartouches (1) et muni de moyens de perçage desdites poches (16) contenant respectivement le conjugué et le substrat ainsi que de moyens de rupture dudit opercule frangible (19′).

2. Dispositif selon la revendication 1, caractérisé par le fait que ledit dispositif de traitement est associé à au moins une pipette de remplissage automatique en sérum des cuves correspondantes desdites cartouches.

3. Dispositif selon l'une des revendications 1 et 2, caractérisé par le fait que ledit dispositif de traitement comporte en outre un module de chargement automatique (101) d'une cartouche dans chaque ascenseur.

4. Dispositif selon l'une des revendications 1 à 3, caractérisé par le fait que ledit dispositif de traitement comporte en outre un module de déchargement automatique des cartouches (103).

5. Dispositif selon l'une des revendications précédentes, caractérisé par le fait que ledit dispositif de traitement comporte un module muni de moyens (83) pour s'appliquer contre ladite poche souple (10) et assurer une homogénéisation des liquides qu'elle contient.

6. Dispositif selon la revendication 1, caractérisé par le fait que l'axe vertical (29) dudit écouvillon (25) est solidaire d'une petite étoile métallique (26) susceptible de coopérer avec un module (81) dudit dispositif de traitement muni d'un électro-aimant situé au droit de la trajectoire dudit axe vertical, afin de faire tourner régulièrement ledit écouvillon sur lui-même.

7. Dispositif selon la revendication 6, caractérisé par le fait que ledit module responsable de la mise en mouvement dudit écouvillon (25) comporte en outre un électro-aimant susceptible de coopérer avec le couvercle ou le fond dudit puits extérieurement métallisé, afin de faire tourner ledit puits autour de son axe vers une première position où son bec verseur est orienté en direction dudit trou d'évacuation (30) et vers une seconde position où son bec verseur est orienté vers le conduit (19) aboutissant à ladite poche souple (10).

8. Dispositif selon l'une des revendications précédentes, caractérisé par le fait que ledit module (82) situé au-dessus de la trajectoire desdites cartouches (1) comporte des marteaux (91, 92) commandables par des électro-aimants, susceptibles d'aplatir lesdites poches (16), un organe coupant (15) étant prévu entre le fond de ces poches et la cuve qui les contient, et qu'il comporte également un marteau (97) susceptible de casser ledit opercule frangible (19′).

9. Dispositif selon l'une des revendications précédentes, caractérisé par le fait que chaque calibre de lecture (65) est une pièce transparente présentant sensiblement la forme d'une gouttière de manière à bloquer radialement ladite poche souple (10), lesdits moyens de blocage de ladite cartouche dans lesdits ascenseurs étant alors débrayés, ladite gouttière ayant deux parois latérales (67, 68) horizontales d'écartement calibré contre lesquelles ladite poche souple vient se plaquer au cours d'une centrifugation.

## Claims

1. A device for performinmg biological analyses by immuno-enzymatic detection of antibodies or antigens in a serum, the device being characterized in that it comprises a processing device for simultaneously processing a plurality of cartridges (1), the assembled device and cartridges being disposed in a thermostatically controlled enclosure, said processing device comprising:
a hub (41) with a motor (43) having an angle encoder for controlling rotation cycles of the hub and capable both of imparting rapid motion thereto for centrifuging, and of imparting a slow step-by-step motion thereto;
a plurality of cartridge-carrying lifts (51) disposed radially on the hub (41) and means for passing each of them from a high, "cartridge-loading" position to a low, "working" position, each of these lifts having an open peripheral face, and a top face and a bottom face which are largely open, and each lift including declutchable means for radially locking the corresponding cartridge for centrifuging;
a plurality of reading gauges (65) fixed to said hub, level with respective peripheral faces of said lifts;
the device also being characterized in that each cartridge (1) is generally rectangular in shape comprising a bottom (2) of thermoformed plastic material covered with a thin transparent sheet of plastic material and thus constituting:
a collecting cuvette (6) disposed in a central position and connected by a duct (7) to a serum storage cuvette (3) situated at the end of the cartridge which is intended to be located adjacent to said hub;
at least two cuvettes (4, 5) between the collecting cuvette and said serum storage cuvette, said at least two cuvettes being connected via respective ducts (8, 9) to suid collecting cuvette (6), and being intended respectively to contain a sachet of conjugate liquid and a sachet of substrate liquid, said collecting cuvette having a small liquid-evacuation hole (30) in its bottom and containing a substantially cylindrical well (20) closed by a lid, and rotatable about a vertical axis (28), with a portion of the side wall of the well forming a pouring or receiving spout (27) for all of the liquids that may flow in the above-mentioned ducts, and which contains a brush (25) having a large surface area per unit volume and which is rotatable about a vertical axis (29) independently of said well, with the bristles of said brush carrying antigens; and
a flexible pocket (10) for containing a diluting or blocking liquid and connected by a duct (19) to said collecting cuvette, and situated close to the open peripheral face of the corresponding lift, said duct being closed by a breakable capsule (19′);
the processing device further comprising at least one peripheral read module (80) situated on the trajectory of said gauges and a module (82) situated above the trajectory of said cartirdges and provided with means (16) for rupturing said sachets respectively containing the conjugate and the substrate, and also with means for breaking said breakable capsule (19′).

2. A device according to claim 1, characterized in that said processing device is associated with at least one pipette for automatically filling the corresponding cuvettes of said cartridges with serum.

3. A device according to claim 1 or 2, characterized in that said processing device also includes a module (101) for automatically loading a cartridge into each lift.

4. A device according to any one of claims 1 to 3, characterized in that said processing device further includes a module (103) for automatically unloading cartridges.

5. A device according to any preceding claim, characterized in that said processing device includes a module provided with means (83) for pressing against said flexible pocket (10) and ensuring that the liquids contained therein are homogenized.

6. A device according to claim 1, characterized in that the vertical axis (29) of said brush (25) is fixed to a small metal star (26) suitable for co-operating with a module (81) of said processing device and provided with an electromagnet situated level with the trajectory of said vertical axis in such a manner as to cause the brush to rotate regularly about its axis.

7. A device according to claim 6, characterized in that the module for moving said brush (25) also includes an electromagnet suitable for co-operating with the lid or the bottom of said well having external metalization in order to rotate said well about its axis towards a first position where the pouring spout points to said evacuation hole (30) and towards a second position where the pouring spout points towards the duct (19) leading to said flexible pocket (10).

8. A device according to any preceding claim, characterized in that said module (12) situated over the trajectory of said cartridges (1) includes hammers (91, 92) controllable by electromagnets and suitable for flattening said sachets (16), with a cutting member (15) being provided between the bottoms of the sachets and the cuvettes in which they are received, said module also including a hammer (97) suitable for breaking said breakable capsule (19′).

9. A device according to any preceding claim, characterized in that each reading gauge (65) is constituted by a transparent member substantially in the form of a gutter serving to lock said flexible pocket (10) radially in position, said means for locking the cartridges in their lifts then being disengaged, said gutter having two horizontal side walls (67, 68) at a calibrated distance apart against which said flexible pocket bears while centrifuging is taking place.

## Patentansprüche

1. Vorrichtung zur Durchführung biologischer Analysen durch immuno-enzymatische Feststellung von Antikörpern oder Antigenen in einem Serum, dadurch gekennzeichnet, daß sie eine Vorrichtung zur gleichzeitigen Bearbeitung einer Vielzahl von Kartuschen (1) besitzt, die in einem temperaturstabilisierten Gehäuse untergebracht ist, wobei die Bearbeitungsvorrichtung aufweist:
- eine Nabe (41), die einem Motor (43) mit Winkelkodierer zugeordnet ist, welcher die Drehzyklen der Nabe zu messen erlaubt und die Nabe in schnelle Bewegung für ein Zentrifugieren oder in eine langsame Bewegung, Schritt für Schritt, versetzen kann,
- eine Vielzahl von Kartuschenträgeraufzügen (51), die radial auf der Nabe (41) befestigt sind, und Mittel, mit denen die Aufzüge von einer hohen Stellung zum Laden einer Kartusche in eine tiefe Arbeitsstellung gebracht werden, wobei jeder Aufzug eine periphere offene Seite, eine Oberseite und eine stark ausgeschnittene Unterseite sowie entkoppelbare Mittel zur radialen Blockierung seiner Kartusche im Fall des Zentrifugierens besitzt,
- eine Vielzahl von Lesekalibern (65), die an der Nabe gegenüber einer peripheren Seite jedes Aufzugs befestigt sind, und weiter dadurch gekennzeichnet, daß jede allgemein rechteckige Kartusche (1) einen Boden (2) aus wärmegeformtem Kunststoff und eine dünne durchscheinende Deckfolie aus Kunststoffmaterial besitzt und wobei der Boden folgende Abteile besitzt:
- ein Empfangsfach (6), das in einer zentralen Position liegt und über einen Kanal (7) an ein Serumlagerfach (3) angeschlossen ist, das an demjenigen Ende der Kartusche liegt, das der Nabe zugewandt ist,
- mindestens zwei Fächer (4, 5), die sich zwischen dem Empfangsfach und dem Serumlagerfach befinden und über Kanäle (8, 9) an das Empfangsfach (6) angeschlossen sind und dazu bestimmt sind, einen Beutel mit konjugierter Flüssigkeit bzw. einen Beutel mit Substratflüssigkeit zu enthalten, wobei das Empfangsfach ein kleines Entleerungsloch (30) für Flüssigkeit in seinem Boden sowie ein im wesentlichen zylindrisches Gefäß besitzt, das mit einem Deckel verschlossen ist, um eine senkrechte Achse (28) drehbeweglich ist und an einem Teil seiner Seitenwand eine Ausguß- oder Eingießnase (27) für alle Flüssigkeiten besitzt, die durch die erwähnten Kanäle gelangen können, wobei das Gefäß eine Bürste (25) mit großer spezifischer Oberfläche besitzt, die um eine senkrechte Achse (29) unabhängig von dem Gefäß in Drehung versetzt werden kann, wobei die Borsten der Bürste Antigenträger sind,
- eine weiche Tasche (10), die eine Blockier- oder Verdünnungsflüssigkeit enthalten kann und über einen Kanal (19) an das Empfangsfach angeschlossen ist, sowie sich in der Nähe der peripheren offenen Seite des entsprechenden Aufzugs befindet, wobei dieser Kanal durch eine zerbrechbare Membran (19′) verschlossen ist,
wobei die Bearbeitungsvorrichtung außerdem mindestens einen peripheren Lesemodul (80), der im Weg der Kaliber (65) liegt, und einen Modul (82) besitzt, der oberhalb des Wegs der Kartuschen (1) liegt und Mittel, um die Beutel (16) zu durchstoßen, die die konjugierte Flüssigkeit bzw. die Substratflüssigkeit enthalten, sowie weiter Mittel zum Zerbrechen der zerbrechbaren Membran (19′) aufweist.

2. Vorrichtung nach Anspruch 1, dadurch gekennzeichnet, daß die Bearbeitungsvorrichtung mindestens einer Pipette zum automatischen Einfüllen von Serum in die entsprechenden Fächer der Kartuschen zugeordnet ist.

3. Vorrichtung nach einem der Ansprüche 1 und 2, dadurch gekennzeichnet, daß die Bearbeitungsvorrichtung außerdem einen Modul (101) zum automatischen Laden einer Kartusche in jeden Aufzug enthält.

4. Vorrichtung nach einem der Ansprüche 1 bis 3, dadurch gekennzeichnet, daß die Bearbeitungsvorrichtung weiter einen Modul (103) zum automatischen Entladen der Kartuschen enthält.

5. Vorrichtung nach einem der vorhergehenden Ansprüche, dadurch gekennzeichnet, daß die Bearbeitungsvorrichtung einen Modul aufweist, der Mittel (83) besitzt, die gegen die weiche Tasche (10) drücken können, um eine homogene Mischung der sie enthaltenden Flüssigkeiten herzustellen.

6. Vorrichtung nach Anspruch 1, dadurch gekennzeichnet, daß die senkrechte Achse (29) der Bürste (25) fest mit einem kleinen Metallstern (26) verbunden ist, der mit einem Modul (81) der Bearbeitungsvorrichtung zusammenwirken kann, welcher einen Elektromagneten gegenüber der Bahn dieser senkrechten Achse aufweist, um die Bürste gleichmäßig um ihre Achse drehen zu lassen.

7. Vorrichtung nach Anspruch 6, dadurch gekennzeichnet, daß der für die Bewegung der Bürste (25) zuständige Modul weiter einen Elektromagneten besitzt, der mit dem Deckel oder dem Boden des außen metallisierten Gefäßes zusammenwirkt, um das Gefäß um seine Achse in eine erste Stellung zu drehen, in der die Ausgußnase zum Entleerungsloch (30) hinweist, oder in eine zweite Stellung, in der die Ausgußnase zu dem bei der weichen Tasche (10) endenden Kanal (19) hinweist.

8. Vorrichtung nach einem der vorhergehenden Ansprüche, dadurch gekennzeichnet, daß der oberhalb der Bahn der Kartuschen (1) liegende Modul (82) Hämmer (91, 92) besitzt, die von Elektromagneten gesteuert werden können und die Beutel (16) flachdrücken können, wobei ein Schneideorgan (15) zwischen dem Boden dieser Beutel und dem sie enthaltenden Fach vorgesehen ist, wobei dieser Modul wetter einen Hammer (97) besitzt, der die zerbrechbare Membran zerbrechen kann.

9. Vorrichtung nach einem der vorhergehenden Ansprüche, dadurch gekennzeichnet, daß jedes ablesbare Kaliber (65) aus einem durchscheinenden Element besteht, das die allgemeine Form einer Rinne besitzt, so daß die weiche Tasche (10) radial blockiert wird, wobei die Blockiermittel in den Aufzügen dann gelöst sind, und daß die Rinne zwei waagrechte Seitenwände (67, 68) in geeichtem Abstand besitzt, gegen die die weiche Tasche sich beim Zentrifugieren anlegt.
